# EUROPEAN PATENT SPECIFICATION

(11) **EP 1 272 215 B1**
(45) Date of publication and mention of the grant of the patent: **10.10.2007**
(21) Application number: 01945007.1
(22) Date of filing: 17.04.2001
(51) Int. Cl.: A61K 39/00, A61P 37/00

(54) **PHARMACEUTICAL PREPARATIONS COMPRISING MODIFIED PEPTIDES**
MODIFIZIERTE PEPTIDE ENTHALTENDE PHARMAZEUTISCHE PRÄPARATIONEN
PREPARATIONS PHARMACEUTIQUES CONTENANT DES PEPTIDES MODIFIES

(30) Priority: 14.04.2000 AT 6572000
(43) Date of publication of application: 08.01.2003
(73) Proprietor: Intercell AG, 1030 Vienna (AT)
(72) Inventor: MATTNER, Frank, A-1190 Vienna (AT); ZAUNER, Wolfgang, A-1030 Vienna (AT); SCHMIDT, Walter, A-1030 Vienna (AT); BUSCHLE, Michael, CH-2300 La Chaux-de-Fonds (CH)
(74) Representative: Alge, Daniel
(86) International application number: PCT/EP2001/004313
(87) International publication number: WO 2001/078767

(56) References cited:
- WO-A-96/37107
- WO-A-97/06821
- WO-A-97/40852

### SEQUENCE LISTING

<110> CISTEM BIOTECHNOLOGIES GMBH
<120> Pharmaceutical preparations comprising modified peptides
<130> R37892
<140>
   <141>
<160> 11
<170> PatentIn Ver. 2.1
<210> 1
   <211> 8
   <212> PRT
   <213> Artificial Sequence
<220>
   <223> Description of Artificial Sequence:peptide
<400> 1
   Val Tyr Asp Phe Phe Val Trp Leu
    1               5
<210> 2
   <211> 36
   <212> PRT
   <213> Artificial Sequence
<220>
   <223> Description of Artificial Sequence:peptide
<220>
   <221> MOD_RES
   <222> (22)
   <223> Orn
<400> 2
   Arg Leu Ala Gly Leu Leu Arg Lys Gly Gly Glu Lys Ile Gly Glu Lys
   1 5 10 15
   Leu Lys Lys Ile Gly Xaa Lys Ile Lys Asn Phe Phe Gln Lys Leu Val
   20 25 30
   Pro Gln Pro Glu
   35
<210> 3
   <211> 8
   <212> PRT
   <213> Artificial Sequence
<220>
   <223> Description of Artificial Sequence:peptide
<400> 3
   Ser Ile Ile Asn Phe Glu Lys Leu
   1 5
<210> 4
   <211> 9
   <212> PRT
   <213> Artificial Sequence
<220>
   <223> Description of Artificial Sequence:peptide
<400> 4
   Phe Ile Ser Ile Asn Phe Glu Lys Leu
   1 5
<210> 5
   <211> 12
   <212> PRT
   <213> Artificial Sequence
<220>
   <223> Description of Artificial Sequence:peptide
<400> 5
   Phe Ile Phe Ile Ser Ile Ile Asn Phe Glu Lys Leu
   1 5 10
<210> 6
   <211> 14
   <212> PRT
   <213> Artificial Sequence
<220>
   <223> Description of Artificial Sequence:peptide
<400> 6
   Phe Ile Phe Ile Phe Ile Ser Ile Ile Asn Phe Glu Lys Leu
   1 5 10
<210> 7
   <211> 15
   <212> PRT
   <213> Artificial Sequence
<220>
   <223> Description of Artificial Sequence:peptide
<400> 7
   Phe Ile Phe Ile Trp Cys Cys Ser Ile Ile Asn Phe Glu Lys Leu
   1 5 10 15
<210> 8
   <211> 10
   <212> PRT
   <213> Artificial Sequence
<220>
   <223> Description of Artificial Sequence:peptide
<400> 8
   Glu Glu Ser Ile Ile Asn Phe Glu Lys Leu
   1 5 10
<210> 9
   <211> 12
   <212> PRT
   <213> Artificial Sequence
<220>
   <223> Description of Artificial Sequence:peptide
<400> 9
   Glu Asp Glu Asp Ser Ile Ile Asn Phe Glu Lys Leu
    1               5                   10
<210> 10
   <211> 12
   <212> PRT
   <213> Artificial Sequence
<220>
   <223> Description of Artificial Sequence:peptide
<400> 10
   Ser Ile Ile Asn Phe Glu Lys Leu Phe Ile Phe Ile
    1               5                   10
<210> 11
   <211> 14
   <212> PRT
   <213> Artificial Sequence
<220>
   <223> Description of Artificial Sequence:peptide
<400> 11
   Leu Ala Leu Ala Leu Ala Ser Ile Ile Asn Phe Glu Lys Leu
    1               5                   10

## Description

The invention relates to pharmaceutical preparations comprising modified peptide antigens, especially peptides suitable for vaccination.

Peptides become increasingly important in modern vaccine design. It has been shown that co-injection of a mixture of poly-L-arginine or poly-L-lysine together with an appropriate peptide as a vaccine protect animals from tumor growth in mouse models (Buschle et al., Gene Ther Mol Biol 1 (1998) 309-321); Schmidt et al., PNAS 94 (1997), 3262-3267). This chemically defined vaccine is able to induce high numbers of antigen/peptide-specific T cells. In order to induce antigen-specific T cells, peptides need to be taken up by antigen presenting cells (APC). These APCs induce an immune cascade eventually leading to the induction of antigen-specific immune effector cells, for example cytotoxic T cells. Antigenic peptides have to form a "depot" allowing APCs sufficient time to infiltrate the (vaccine) injection site. Unfortunately, most peptides fail to form such a depot, even when injected together with an adjuvant. It is a well recognized problem in the art that many promising peptides defining antigenic regions of medically important pathogens fail to provide a sufficient immune response *in vivo.*

WO 97/40852 relates to a method of improving the binding affinity of a peptide epitope for MHC class II molecules by attaching to the N-terminus of the peptide epitope a hydrophobic amino acid sequence. This modified peptide shows an improved capability of binding to MHC class II molecules whereby these so formed complexes are used to inactivate T cells bearing receptors that recognize an epitope on the modified peptide. The formed complexes can therefore be used therapeutically for inactivating unwanted immune responses, e.g. autoimmune responses. Therefore the aim of the modified peptides according to the WO 97/40852 is not an enhanced uptake in antigen presenting cells, but to improve the binding of the peptide epitope to MHC class II molecules and therefore to inactivate T cells. This is contrary to the activation of T cells by antigen presenting cells which take up the antigen, process it and display the processed fragments of the antigen on their cell surface mediated by MHC molecules.

In US 5,726,292 a construct is described comprising a protein, protein fragment, polypeptide or peptide, a hydrophobic anchor and a proteosome, which construct is used as an immunogenic composition for the use as therapeutic agent and vaccine. This construct shows improved immuno potentiating activity. The hydrophobic anchor may be a hydrophobic peptide of about 3 to 50 amino acids in length. It is shown in this document that the peptide comprising the hydrophobic amino acid sequence alone (without a proteosome) was not immunogenic, whereas the same peptide comprising the hydrophobic tail complexed to proteosomes showed high immunogenic activities due to the anchoring in the lipid component.

It is therefore an object of the present invention to provide pharmaceutical preparations comprising peptides which induce a sufficient immune response in a mammal, especially in humans, to which this peptide is applied. It is a further object to provide compositions comprising modified peptides derived from wild type antigens which allow a stronger immune response than the wild type peptide. Further, according to the present invention non-immunogenic peptides being antigens shall be modified to become immunogenic. Yet another object is to improve the immunogenic quality of peptide antigens in pharmaceutical compositions.

These objects are solved by a pharmaceutical preparation comprising peptides of the formula

X_{N} - Peptide_{L} - X_{M},

characterized in that X_{N} and/or X_{M} is
a) selected from (FI)₁₋₅, (FI)₁₋₅W, (FI)₁₋₅W(FI)₁₋₅, (FL)₁₋₅, (FL)₁₋₅W, (FL)₁₋₅W(FL)₁₋₅, (FI)₁₋₅(FL)₁₋₅, (FL)₁₋₅(FI)₁₋₅, F(II)₁₋₅, (FIL)₁₋₅, (FLI)₁₋₅, (FLL)₁₋₅, (FIF)₁₋₅, (FLF)₁₋₅, (WIL)₁₋₅, (WLI)₁₋₅, (LWI)₁₋₅, (IWL)₁₋₅, (WI)₁₋₅, (WL)₁₋₅, (WL)₁₋₅F, (WI)₁₋₅F, (FV)₁₋₅, (FVI)₁₋₅, (FAI)₁₋₅, (FIA)₁₋₅, (FIV)₁₋₅, (FAI)₁₋₅, (FV)₁₋₅C-C(FV)₁₋₅, (FA)₁₋₅C-C (FA)₁₋₅ and (FV)₁₋₅C-C (FA)₁₋₅. (FI)₁₋₅C-C (FI)₁₋₅, (FL)₁₋₅C-C (FL)₁₋₅, (WI)₁₋₅C-C (WI)₁₋₅; (FI)_{1- 5}WC-C, (FI)₁₋₅C-C, (FL)₁₋₅WC-C, (FL)₁₋₅C-C, (WI)₁₋₅C-C, (WI)₁₋₅FC-C, wherein F is Phe, I is Ile, W is Trp, C is Cys, C-C is a cystein bridge, L is Leu, V is Val and A is Ala;
   or
b) from (ED)₁₋₅, (EDE)₁₋₅, (DED)₁₋₅, (DE)₁₋₅, (DEE)₁₋₅, (EED)₁₋₅, (EXE)₁₋₅, (DXD)₁₋₅, (EXD)₁₋₅, (EX)₁₋₅, (DX)_{1-5,} (ED)₁₋₅(FI)₁₋₅ and (FI)₁₋₅(ED)₁₋₅, (ED)₁₋₅C-C (ED)₁₋₅, (ED)₁₋₅C-C, wherein X is selected from Gly, Ala, Val, Leu, Ile, Pro, Phe, Tyr, Trp, Cys and Met and E is Glu, D is Asp, F is Phe, I is Ile, C is Cys and C-C is a cystein bridge;
wherein Peptide_{L} is a potentially immunogenic fragment consisting of L amino acid residues, which may be derived from a naturally occurring protein; alternatively, Peptide_{L} may also be a synthetically designed immunogenic peptide; L is an integer from 6 to 25, X_{N} and X_{M} being amino acid sequences not occurring at this position and in this constellation with Peptide_{L} (i.e. not naturally occuring, if derived from a naturally occuring substance and not described at this position/constellation, if derived from a synthetic peptide design method), and a polycationic substance.

The present inventors have observed that especially hydrophobic peptides are able to induce specific T cells, e.g. the tyrosinase-related protein-2-derived peptide VYDFFVWL. However, many peptides which are antigens, potentially suitable as a vaccine, are not very hydrophobic per se. Therefore, the adding of amino acids according to the present invention at the N- and/or C-terminus to a known (non-hydrophobic) peptide sequence in combination with a polycationic substance renders such a peptide hydrophobic and therefore more immunogenic. With the present invention it could be shown that the more hydrophobic amino acids were added at the N- and/or the C-terminus of a neutral or hydrophilic antigen, the more peptide specific T cells were induced by such a modified antigen, if these modified peptides are combined with polycationic substances.

In principle any peptide can be modified to become an immunogenic peptide in the composition according to the present invention. It is, however, preferred to modify peptides encoding for antigenic determinants according to the present invention. Especially peptides with a low hydrophobicity or even hydrophilic peptides which - although perhaps coding for an exposed surface determinant of a pathogen - elicit no effective immune response (i.e. are only potentially pathogenic), may be transformed to efficient immunogenics if modified according to the present invention. If the Peptide_{L} is a peptide derived from natural sources by fragmentation of pathogen proteins, the length should be selected suitable to sterically define e.g. the antigenic determinant. Therefore, L is an integer from 6 to 25, especially from 8 to 25.

Preferably, the amino acid residues are connected via peptide bonds with Peptide_{L} and with each other. Such peptides may easily and economically be produced by recombinant expression or Merri-field solid phase techniqes. These methods lead to peptide-bond linked amino acid chains. However, other covalent chemical connections between the single amino acid residues are also possible, especially disulfide bonds (C-C) or other connections wherein functional groups of the amino acid residues are affected. In the present specification (if not otherwise indicated) amino acid residues are linked via peptide bonds (i.e. "CC" means C bond via peptide bond to C; "C-C" means C bound via disulfide bound to C).

On the other hand, it could also be shown that the addition of negatively charged amino acids at the N- and/or the C-terminus of a known peptide also leads to improved immunogenic properties, to a "depot" formation.

Therefore, the peptides to be used in the composition according to the present invention have X_{N} and/or X_{M} selected from (FI)₁₋₅, (FI)₁₋₅W, (FI)₁₋₅W(FI)₁₋₅, (FL)₁₋₅, (FL)₁₋₅W, (FL)₁₋₅W(FL)₁₋₅, (FI)₁₋₅(FL)₁₋₅, (FL)₁₋₅(FI)₁₋₅, F(II)₁₋₅, (FIL)₁₋₅, (FLI)₁₋₅, (FLL)₁₋₅, (FIF)₁₋₅, (FLF)₁₋₅, (WIL)₁₋₅, (WLI)₁₋₅, (LWI)₁₋₅, (IWL)₁₋₅, (WI)₁₋₅, (WL)₁₋₅, (WL)₁₋₅F, (WI)₁₋₅F, (FV)₁₋₅, (FVI)₁₋₅, (FAI)₁₋₅, (FIA)₁₋₅, (FIV)₁₋₅, (FAI)₁₋₅, (FV)₁₋₅C-C (FV)₁₋₅, (FA)₁₋₅C-C (FA)₁₋₅ and
(FV)₁₋₅C-C (FA)₁₋₅, (FI)₁₋₅C-C (FI)₁₋₅, (FL)₁₋₅C-C (FL)₁₋₅, (WI)₁₋₅C-C (WI)₁₋₅; (FI)₁₋₅WC-C, (FI)₁₋₅C-C, (FL)₁₋₅WC-C, (FL)₁₋₅C-C, (WI)₁₋₅C-C, (WI)₁₋₅FC-C, wherein F is Phe, I is Ile, W is Trp, C is Cys, C-C is a cystein bridge, L is Leu, V is Val and A is Ala.

Peptides with negatively charged "tails" are peptides, wherein X_{N} and/or X_{M} is selected from (EO)₁₋₅, (EOE)₁₋₅, (DED)₁₋₅, (OE)₁₋₅, (DEE)₁₋₅, (EEO)₁₋₅, (EXE)₁₋₅, (DXD)₁₋₅, (EXD)₁₋₅, (EX)₁₋₅, (DX)₁₋₅, (ED)₁₋₅(FI)₁₋₅ and (FI)₁₋₅(ED)₁₋₅, (EO)₁₋₅C-C(ED)₁₋₅, (ED)₁₋₅C-C, wherein X is selected from Gly, Ala, Val, Leu, Ile, Pro, Phe, Tyr, Trp, Cys and Met and E is Glu, D is Asp, F is Phe, I is Ile, C is Cys and C-C is a cystein bridge.

The present pharmaceutical preparations are especially suitable for vaccination. Therefore, the present invention is also drawn to a vaccine comprising a pharmaceutical preparation according to the present invention, optionally with further pharmaceutically acceptable carrier substances, especially adjuvants. Primary adjuvants in the composition according to the present invention are, of course, polycationic substances which are especially suited when negatively charged amino acid "tails" are used as X_{N} and/or X_{M}.

The polycationic compound(s) to be used according to the present invention may be any polycationic compound which shows the characteristic effects according to the WO 97/30721. Preferred polycationic compounds are selected from basic polypeptides, organic polycations, basic polyamino acids or mixtures thereof. These polyamino acids should have a chain length of at least 4 amino acid residues (see: Tuftsin as described in Goldman et al. (1983)). Especially preferred are substances like polylysine, polyarginine and polypeptides containing more than 20%, especially more than 50% of basic amino acids in a range of more than 8, especially more than 20, amino acid residues or mixtures thereof. Other preferred polycations and their pharmaceutical compositons are described in WO 97/30721 (e.g. polyethyleneimine) and WO 99/38528. Preferably these polypeptides contain between 20 and 500 amino acid residues, especially between 30 and 200 residues.

These polycationic compounds may be produced chemically or recombinantly or may be derived from natural sources. Cationic (poly)peptides may also be anti-microbial with properties as reviewed in Ganz et al, 1999; Hancock, 1999. These (poly)peptides may be of prokaryotic or animal or plant origin or may be produced chemically or recombinantly (Andreu et al., 1998; Ganz et al., 1999; Simmaco et al., 1998). Peptides may also belong to the class of defensins (Ganz, 1999; Ganz et al., 1999). Sequences of such peptides can be, for example, be found in the Antimicrobial Sequences Database under the following internet address:
http://www.bbcm.univ.trieste.it/-tossi/paql.html

Such host defence peptides or defensives are also a preferred form of the polycationic polymer according to the present invention. Generally, a compound allowing as an end product activation (or down-regulation) of the adaptive immune system, preferably mediated by APCs (including dendritic cells) is used as polycationic polymer.

Especially preferred for use as polycationic substance in the present invention are cathelicidin derived antimicrobial peptides or derivatives thereof (WO 02/13857), especially antimicrobial peptides derived from mammal cathelicidin, preferably from human, bovine or mouse.

Polycationic compounds derived from natural sources include HIV-REV or HIV-TAT (derived cationic peptides, antennapedia peptides, chitosan or other derivatives of chitin) or other peptides derived from these peptides or proteins by biochemical or recombinant production. Other preferred polycationic compounds are cathelin or related or derived substances from cathelin. For example, mouse cathelin is a peptide which has the amino acid sequence NH₂-RLAGLLRKGGEKIGEKLKKIGOKIKNFFQKLVPQPE-COOH. Related or derived cathelin substances contain the whole or parts of the cathelin sequence with at least 15-20 amino acid residues. Derivations may include the substitution or modification of the natural amino acids by amino acids which are not among the 20 standard amino acids. Moreover, further cationic residues may be introduced into such cathelin molecules. These cathelin molecules are preferred to be combined with the antigen. These cathelin molecules surprisingly have turned out to be also effective as an adjuvant for a antigen without the addition of further adjuvants. It is therefore possible to use such cathelin molecules as efficient adjuvants in vaccine formulations with or without further immunactivating substances.

Another preferred polycationic substance to be used according to the present invention is a synthetic peptide containing at least 2 KLK-motifs separated by a linker of 3 to 7 hydrophobic amino acids (WO 02/32451).

Immunostimulatory deoxynucleotides are e.g. natural or artificial CpG containing DNA, short stretches of DNA derived from non-vertebrates or in form of short oligonucleotides (ODNs) containing non-methylated cytosine-guanine di-nucleotides (CpG) in a certain base context (e.g. Krieg et al., 1995) but also inosine containing ODNs (I-ODNs) as described in WO 01/93905 or poly I:C and oligo dI:C as described in WO 01/93903.

Neuroactive compounds, e.g. combined with polycationic substances are described in WO 01/24822.

The vaccine compositions according to the present invention may be formulated according to known methods, e.g. as i.v. vaccines, DNA vaccines, oral vaccines, transdermal vaccines, topical vaccines, intranasal vaccines and as combination vaccines. The dosages may be selected by standard processes. For vaccines which are improvements of known vaccines, however, a lower dosage than the known vaccine is possible for the same protection and therefore preferred.

Preferably, the vaccine is provided in a storage stable form, e.g. lyophilized, optionally provided in combination with a suitable reconstitution solution.

The invention will be described in more detail by the following examples and the drawing figures, but would of course not be restricted thereto.
Fig.1 shows the immunization with an ovalbumin derived peptide with a hydrophobic tail and (optionally) polyarginine.
Fig.2 shows the immunization with an ovalbumin derived peptide and a negatively charged amino acid tail with polyarginine.
Fig.3 shows the immunization with an ovalbumin derived, class I H-2K^{b}-restricted peptide which was made "more hydrophobic" by directly adding F and I at the C-terminus.
Fig.4 shows the immunization with an ovalbumin peptide which was made "more hydrophobic" by directly adding L and A at the N-terminus.

### Example 1:

The ovalbumin derived class I H-2K^{b}, restricted peptide ("245"), SIINFEKL (which is a weak inducer of T cells, alone or in combination with an adjuvant like poly-L-arginine) was made "more hydrophobic by directly adding F and I or by adding FIFIW via a C-C bridge. Groups of 4 mice (C75BL/6, femal, 8 weeks of age, H-2^{b} were injected subcutaneoulsy in the flank 3 times (days 0, 21 and 28) as follows (dose 100 µg of peptide, molarity adapted; +/- 100 µg poly-L-arginine per mouse):
1) SIINFEKL (peptide "245", 4 of 8 amino acids (aa) hydrophobic)
2) FISIINFEKL (6 of 10 aa hydrophobic)
3) FIFISIINFEKL (8 of 12 aa hydrophobic)
4) FIFIFISIINFEKL (10 of 14 aa hydrophobic)
5) SIINFEKL + poly-L-arginine
6) FISIINFEKL + poly-L-arginine
7) FIFISIINFEKL + poly-L-arginine
8) FIFIFISIINFEKL + poly-L-arginine
9) FIFIWC-CSIINFEKL (11 of 15 aa hydrophobic)
10) FIFIWC-CSIINFEKL + poly-L-arginine

One week after the third vaccination spleens were removed and the splenocytes were activated *ex vivo* with peptide SIINFEKL ("245") to determine IFN-γ-producing specific cells (ELISpot assay).

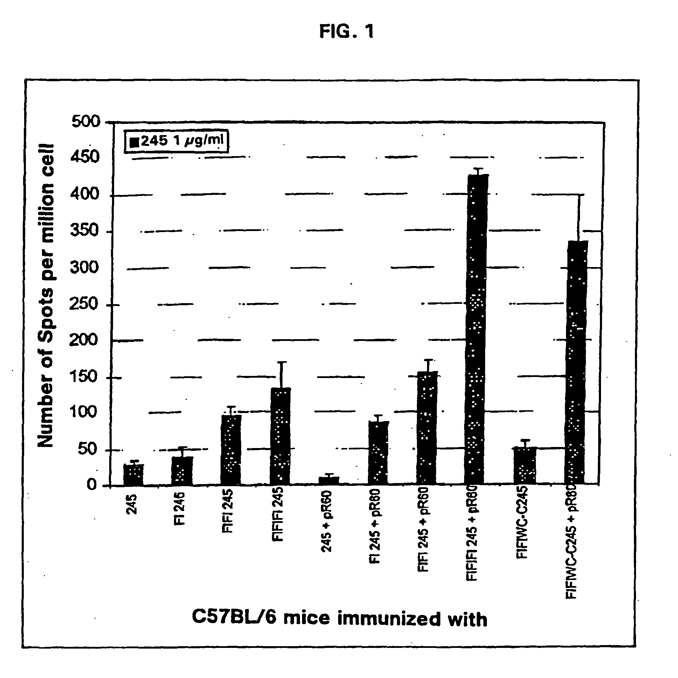

The more hydrophobic amino acids were added in the N-terminus of peptide SIINFEKL, the more peptide specific T cells were induced (see Fig.1). Peptides FIFISIINFEKL and FIFIFISIINFEKL were even able to induce about 100 peptide specific T cells per 1 million spleen cells without adding poly-L-arginine. Peptide FIFIFISIINFEKL in combination with poly-L-arginine induced more than 400 SIINFEKL specific T cells among 1 million splenocytes (similar to peptide FIFIWC-CSIINFEKL).

### Example 2:

The neutral peptide SIINFEKL (one negatively charged (E), one positively charged (K) amino acid) was rendered negative by adding (at the N-terminus) EE or EDED, respectively.

Groups of 4 mice (C57BL/6, female, 6 weeks of age, II-2^{b}) were injected subcutaneously in the footpad 3 times (days 0, 14, 28) as follows (dose 100 µg of peptide, molarity adapted; +100 µg poly-L-arginine per mouse):
1) SIINFEKL (peptide "245") + poly-L-arginine
2) EESIINFEKL (peptide "246") + poly-L-arginine
3) EDEDSIINFEKL (peptide "080") + poly-L-arginine
Groups of 4 mice were vaccinated subcutaneously in the footpad 3 times as indicated. One week after the 3rd vaccination, popliteal lymph nodes and spleens were removed and lymph node cells and splenocytes were activated *ex vivo* with peptide SIINFEKL ("245") to determine IFN-γ-producing specific cells (ELISpot assay).

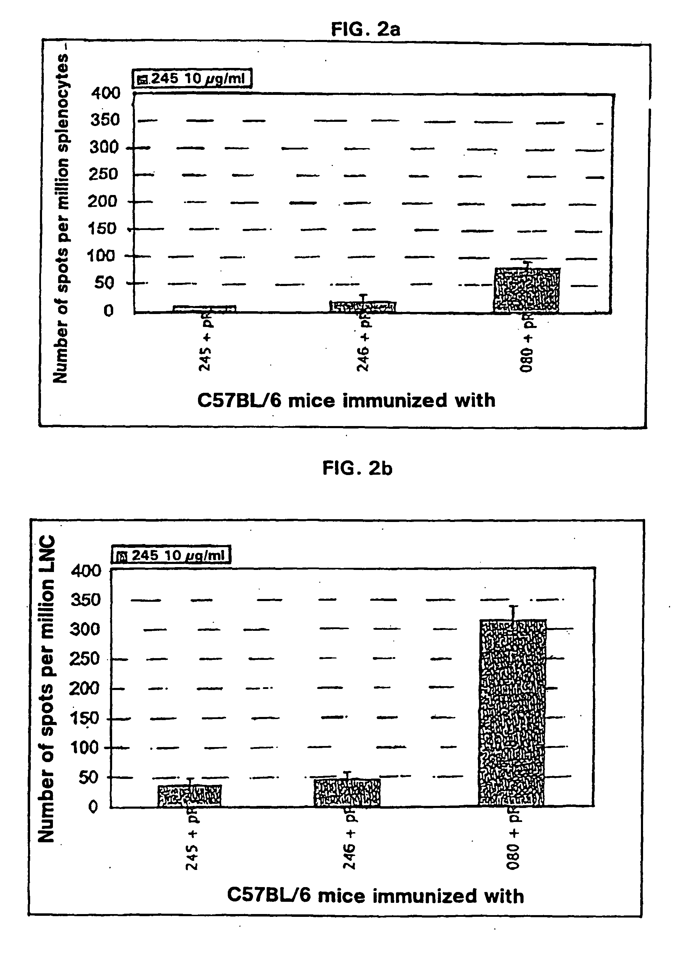

As can be seen in Fig.2, the addition of 4 negatively-charged amino acids (EDED) at the N-terminus of peptide SIINFEKL makes this peptide (in combination with poly-L-arginine) able to induce a high amount of specific IFN-γ-producing T cells in the draining (popliteal) lymph node (local response) and in the spleen (systemic response).

Thus, the addition of hydrophobic amino acids as well as the addition of negatively charged amino acids transforms the peptide SIINFEKL to a good inducer of specific T cells.

### Example 3:

### C-terminus instead of N-terminus

SIINFEKL with 4 hydrophobic (bold) amino acids was made "more hydrophobic" by directly adding F and I at the C-terminus. The already described (N-terminus-prolonged) peptide FIFISIINFEKL served as control for SIINFEKLFIFI.

Groups of 4 mice (C57BL/6, female, 8 weeks of age, H-2^{b}) were injected subcutaneously into the hind footpads as follows (dose 100 µg of peptide, molarity adapted; + 100 µg poly-L-arginine per mouse):
1) SIINFEKL 8peptide "245") + poly-L-arginine (4 of 8 aa hydrophobic)
2) FIFISIINFEKL (peptide "1015") + poly-L-arginine (8 of 12)
3) SIINFEKLFIFI (peptide "1078") + poly-L-arginine (8 of 12)

Groups of 4 mice were vaccinated subcutaneously in the hind footpads as indicated. 7 days after the vaccination single cell suspensions of spleens were prepared and cells were restimulated ex vivo to detect IFN-γ-producing cells via ELISpot assay.

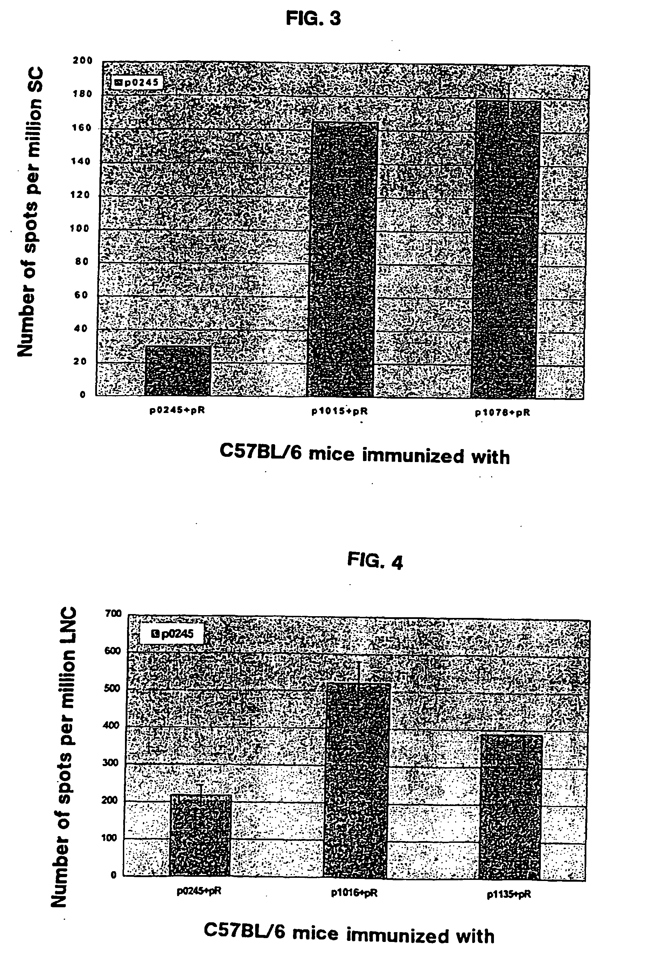

As can be seen in Fig.3 in contrast to peptide SIINFEKL, the peptides FIFISIIFEKL and SIINFEKLFIFI (in combination with poly-L-arginine) were able to induce high numbers of SIINFEKL-specific IFN-γ-producing T cells.

Thus, the addition of hydrophobic amino acids works equally well (with regard to induction of peptide-specific T cells) at the N-terminus or C-terminus, respectively.

### Example 4:

### L and A instead of F and I

The peptide SIINFEKL with 4 hydrophobic (bold) amino acids was made "more hydrophobic" by directly adding L and A at the N-terminus. The already described (N-terminus-prolonged) peptide FIFIFISIINFEKL served as control for the peptide LALALASIINFEKL.

Groups of 4 mice (C57BL/6, female, 8 weeks of age, H-2^{b}) were injected subcutaneously into the hind footpads as follows (dose 100µg of peptide, molarity adapted; + 100µg poly-L-arginine per mouse):
1) SIINFEKL (peptide "245") + poly-L-arginine
2) FIFIFISIINFEKL (peptide "1016") + poly-L-arginine
3) LALALASIINFEKL (peptide "1135") + poly-L-arginine

The result of the vaccination experiment with the peptide SIINFEKL which was made "more hydrophobic" by directly adding L and A at the N-terminus is shown in Fig.4.

## Claims

1. Pharmaceutical preparation comprising a peptide of the formula
X_{N} - Peptide_{L} - X_{M},
**characterized in that** X_{N} and/or X_{M} is
a) selected from (FI)₁₋₅, (FI)₁₋₅W, (FI)₁₋₅W(FI)₁₋₅, (FL)₁₋₅, (FL)₁₋₅W, (FL)₁₋₅W(FL)₁₋₅, (FI)₁₋₅(FL)₁₋₅, (FL)₁₋₅(FI)₁₋₅, F(II)₁₋₅, (FIL)₁₋₅, (FLI)₁₋₅, (FLL)₁₋₅, (FIF)₁₋₅, (FLF)₁₋₅, (WIL)₁₋₅, (WLI)₁₋₅, (LWI)₁₋₅, (IWL)₁₋₅, (WI)₁₋₅, (WL)₁₋₅, (WL)₁₋₅F, (WI)₁₋₅F, (FV)₁₋₅, (FVI)₁₋₅, (FAI)₁₋₅, (FIA)₁₋₅, (FIV)₁₋₅, (FAI)₁₋₅, (FV)₁₋₅C-C (FV)₁₋₅, (FA)₁₋₅C-C (FA)₁₋₅ and (FV)₁₋₅C-C (FA)₁₋₅, (FI)₁₋₅C-C(FI)₁₋₅, (FL)₁₋₅C-C(FL)₁₋₅, (WI)₁₋₅C-C(WI)₁₋₅; (FI)₁₋₅WC-C, (FI)₁₋₅C-C, (FL)₁₋₅WC-C, (FL)₁₋₅C-C, (WI)₁₋₅C-C, (WI)₁₋₅FC-C, wherein F is Phe, I is Ile, W is Trp, C is Cys, C-C is a cystein bridge, L is Leu, V is Val and A is Ala; or
b) from (ED)₁₋₅, (EDE)₁₋₅, (DED)₁₋₅, (DE)₁₋₅, (DEE)₁₋₅, (EED)₁₋₅, (EXE)₁₋₅, (DXD)₁₋₅, (EXD)₁₋₅, (EX)₁₋₅, (DX)₁₋₅, (ED)₁₋₅(FI)₁₋₅ and (FI)₁₋₅(ED)₁₋₅, (ED)₁₋₅C-C(ED)₁₋₅, (ED)₁₋₅C-C, wherein X is selected from Gly, Ala, Val, Leu, Ile, Pro, Phe, Tyr, Trp, Cys and Met and E is Glu, D is Asp, F is Phe, I is Ile, C is Cys and C-C is a cystein bridge;
wherein Peptide_{L} is a potentially immunogenic fragment consisting of L amino acid residues; L is an integer from 6 to 25, with the proviso that X_{N} and X_{M} are amino acid sequences not naturally occuring at this position with Peptide_{L} if derived from a naturally occuring substance and not described at this position/constellation with peptide, if derived from a synthetic peptide design method, and a polycationic substance.

2. Pharmaceutical preparation according to claim 1, **characterized in that** the polycationic substance is selected from the group consisting of basic polypeptides, organic polycations and polycationic antimicrobial peptides, especially cathelicidin derived antimicrobial peptides.

3. Vaccine comprising a pharmaceutical preparation according to claim 1 or 2.

## Patentansprüche

1. Pharmazeutische Zusammensetzung, umfassend ein Peptid der Formel
X_{N} - Peptid_{L} - X_{M},
**dadurch gekennzeichnet, dass** X_{N} und/oder X_{M} ausgewählt sind aus
a) (FI)₁₋₅, (FI)₁₋₅W, (FI)₁₋₅W (FI)₁₋₅, (FL)₁₋₅, (FL)₁₋₅W, (FL)₁₋₅W (FL)₁₋₅, (FI)₁₋₅(FL)₁₋₅, (FL)₁₋₅(FI)₁₋₅, F(II)₁₋₅, (FIL)₁₋₅, (FLI)₁₋₅, (FLL)₁₋₅, (FIF)₁₋₅, (FLF)₁₋₅, (WIL)₁₋₅, (WLI)₁₋₅, (LWI)₁₋₅, (IWL)₁₋₅, (WI)₁₋₅, (WL)₁₋₅, (WL)₁₋₅F, (WI)₁₋₅F, (FV)₁₋₅, (FVI)₁₋₅, (FAI)₁₋₅, (FIA)₁₋₅, (FIV)₁₋₅, (FAI)₁₋₅, (FV)₁₋₅C-C(FV)₁₋₅, (FA)₁₋₅C-C(FA)₁₋₅ und (FV)₁₋₅C-C(FA)₁₋₅, (FI)₁₋₅C-C(FI)₁₋₅, (FL)₁₋₅C-C(FL)₁₋₅, (WI)₁₋₅C-C(WI)₁₋₅; (FI)₁₋₅WC-C, (FI)₁₋₅C-C, (FL)₁₋₅WC-C, (FL)₁₋₅C-C, (WI)₁₋₅C-C, (WI)₁₋₅FC-C, worin F Phe ist, I Ile ist, W Trp ist, C Cys ist, C-C eine Cysteinbrücke ist, L Leu ist, V Val ist und A Ala ist; oder
b) (ED)₁₋₅, (EDE)₁₋₅, (DED)₁₋₅, (DE)₁₋₅, (DEE)₁₋₅, (EED)₁₋₅, (EXE)₁₋₅, (DXD)₁₋₅, (EXD)₁₋₅, (EX)₁₋₅, (DX)₁₋₅, (ED)₁₋₅(FI)₁₋₅ und (FI)₁₋₅(ED)₁₋₅, (ED)₁₋₅C-C(ED)₁₋₅, (ED)₁₋₅C-C, worin X ausgewählt ist aus Gly, Ala, Val, Leu, Ile, Pro, Phe, Tyr, Trp, Cys und Met und E Glu ist, D Asp ist, F Phe ist, I Ile ist, C Cys ist und C-C eine Cysteinbrücke ist;
wobei Peptid_{L} ein potentiell immunogenes Fragment ist, bestehend aus L-Aminosäureresten; L eine ganze Zahl von 6 bis 25 ist, mit der Maßgabe, dass Xₙ und Xₘ Aminosäuresequenzen sind, die in dieser Position mit Peptid_{L} nicht natürlich vorkommen, wenn aus einer natürlich vorkommenden Substanz abgeleitet und nicht in dieser Position/Konstellation mit Peptid_{L} beschrieben, wenn von einem synthetischen Gestaltungsverfahren für Peptide abgeleitet, und eine polykationische Substanz.

2. Pharmazeutische Zusammensetzung nach Anspruch 1, **dadurch gekennzeichnet, dass** die polykationische Substanz ausgewählt ist aus der Gruppe bestehend aus basischen Polypeptiden, organischen Polykationen und polykationischen antimikrobiellen Peptiden, insbesondere antimikrobielle Peptide, die aus Cathelicidin abgeleitet sind, oder Mischungen davon.

3. Vakzin umfassend eine pharmazeutische Zusammensetzung gemäß Anspruch 1 oder 2.

## Revendications

1. Préparation pharmaceutique comprenant un peptide de formule
X_{N}-Peptide_{L}-X_{M},
**caractérisée en ce que** X_{N} et/ou X_{M} sont
a) choisis parmi (FI)₁₋₅, (FI)₁₋₅W, (FI)₁₋₅W(FI)₁₋₅, (FL)₁₋₅, (FL)₁₋₅W, (FL)₁₋₅W (FL)₁₋₅, (FI)₁₋₅(FL)₁₋₅, (FL)₁₋₅(FI)₁₋₅, F(II)₁₋₅, (FIL)₁₋₅, (FLI)₁₋₅, (FLL)₁₋₅, (FIF)₁₋₅, (FLF)₁₋₅, (WIL)₁₋₅, (WLI)₁₋₅, (LWI)₁₋₅, (IWL)₁₋₅, (WI)₁₋₅, (WL)₁₋₅, (WL)₁₋₅F, (WI)₁₋₅F, (FV)₁₋₅, (FVI)₁₋₅, (FAI)₁₋₅, (FIA)₁₋₅, (FIV)₁₋₅, (FAI)₁₋₅, (FV)₁₋₅C-C (FV)₁₋₅, (FA)₁₋₅C-C (FA)₁₋₅ et (FV)₁₋₅C-C (FA)₁₋₅, (FI)₁₋₅C-C (FI)₁₋₅, (FL)₁₋₅C-C(FL)₁₋₅, (WI)₁₋₅C-C (WI)₁₋₅, (FI)₁₋₅WC-C, (FI)₁₋₅C-C, (FL)₁₋₅WC-C, (FL)₁₋₅C-C, (WI)₁₋₅C-C, (WI)₁₋₅FC-C, où F est Phe, I est Ile, W est Trp, C est Cys, C-C est un pont cystéine, L est leu, V est Val et A est Ala ; ou
b) parmi (ED)₁₋₅, (EDE)₁₋₅, (DED)₁₋₅, (DE)₁₋₅, (DEE)₁₋₅, (EED)₁₋₅, (EXE)₁₋₅, (DXD)₁₋₅, (EXD)₁₋₅, (EX)₁₋₅, (DX)₁₋₅, (ED)₁₋₅(FI)₁₋₅ et (FI)₁₋₅(ED)₁₋₅, (ED)₁₋₅C-C(ED)₁₋₅, (ED)₁₋₅C-C, où X est choisi parmi Gly, Ala, Val, Leu, Ile, Pro, Phe, Tyr, Trp, Cys et Met et E est Glu, D est Asp, F est Phe, I est Ile, C est Cys et C-C est un pont cystéine ;
dans laquelle Peptide_{L} est un fragment potentiellement immunogène composé de résidus d'acides aminés L ; L est un entier de 6 à 25, à condition que X_{N} et X_{M} ne soient pas des séquences d'acides aminés d'origine naturelle au niveau de cette position avec le Peptide_{L} s'il dérive d'une substance d'origine naturelle et ne soient pas décrits au niveau de cette position/constellation avec le Peptide_{L} s'il dérive d'un procédé de synthèse peptidique, et une substance polycationique.

2. Préparation pharmaceutique selon la revendication 1, **caractérisée en ce que** la substance polycationique est choisie dans le groupe constitué par des polypeptides basiques, des polycations organiques et des peptides polycationiques antimicrobiennes, en particulier des peptides antimicrobiens dérivés de la cathélicidine, ou leurs mélanges.

3. Vaccin comprenant une préparation pharmaceutique selon la revendication 1 ou la revendication 2.
